# EUROPEAN PATENT APPLICATION

(11) **EP 4 385 548 A2**
(43) Date of publication of application: **19.06.2024**
(21) Application number: 24169796.0
(22) Date of filing: 17.06.2020
(51) Int. Cl.: A61M 25/09

(54) **AN ENDOSCOPIC RETROGRADE CHOLANGIOPANCREATOGRAPHY (ERCP) CATHETER AND GUIDEWIRE WITH SENSORS AND METHODS OF USING THE SAME**

(30) Priority: 20.06.2019 US 201962863941 P
(62) Divisional of application: 20827067.8
(71) Applicant: Yissum Research Development Company of the Hebrew University of Jerusalem Ltd., 9139002 Jerusalem (IL); Hadasit Medical Research Services and Development Ltd., 9112001 Jerusalem (IL)
(72) Inventor: GHOSHEH, Mohammad, 9139002 Jerusalem (IL); KHALAILEH, Abed, 9112001 Jerusalem (IL); PLOTKIN, Yevgeni, 9112001 Jerusalem (IL); NAHMIAS, Yaakov, 9076739 Mevaseret Zion (IL); MINTZ, Yoav, 9641004 Jerusalem (IL); FRIED, Elchanan, 9330608 Jerusalem (IL)
(74) Representative: Kancelaria Eupatent.pl Sp. z.o.o

(57) **Abstract**

Provided herein is an endoscopic retrograde cholangiopancreatography (ERCP) catheter system which includes a guidewire; an elongated catheter including a distal end configured to be positioned within the common bile duct (CBD) or the pancreatic duct of a subject, and a multiplicity of internal lumens extending from a proximal end of the elongated catheter to a distal end of the elongated catheter opposite thereto, said lumens comprising at least a first lumen having an opening at a tip on the distal end of the catheter, said first lumen is configured to allow passage therethrough of the guidewire; and one or more sensors configured to sense or measure one or more of: electrical impedance, resistance, conductance and electrical signal, at the distal end of the catheter and/or the guidewire.

## Description

The present disclosure relates generally to improved catheter systems, and in particular, improved endoscopic retrograde cholangiopancreatography (ERCP) catheters and guidewires, having multiple ports and sensing elements, as well as methods of using the same.

### BACKGROUND

Endoscopic Retrograde CholangioPancreatography (ERCP) is a diagnostic and therapeutic procedure for various pathologies of the bile and pancreatic systems.

ERCP is performed when the bile or pancreatic ducts have become narrowed or blocked because of, for example, gallstones that form in the gallbladder and obstruct the common bile duct, infection, acute pancreatitis, chronic pancreatitis, trauma or surgical complications in bile or pancreatic ducts, pancreatic pseudocysts, tumors or cancers of the bile ducts, tumors or cancers of the pancreas, and other indications.

The procedure involves insertion of an endoscope through the upper gastrointestinal system into the second part of the duodenum (proximal part of the small intestine). Once the endoscope is in the duodenum, a catheter is passed through the working channel of the endoscope and extends through it. A guidewire is passed through the catheter into the common bile duct (CBD) through the sphincter of Oddi. Sometimes, an incision into the sphincter of Oddi is made at this point. A catheter is passed on top of the guidewire into the CBD, the guidewire is removed, and radiopaque dye is injected through the catheter to visualize the bile ducts.

Although ERCP is considered more beneficial than surgical treatments, various serious complications, including pancreatitis can occur as a result of the procedure. Some of these complications can be attributed to, among others: Injection of contrast material into the pancreatic duct, insertion of a guidewire or other mechanical manipulations inside the pancreatic duct thereby injuring the pancreas, and post-procedural obstruction of the pancreatic duct with a stone.

Thus, there is a need in the art for improved devices and methods to allow a safer ERCP procedure, by reducing mechanical manipulation near the pancreas and avoiding as much as possible injecting contrast into it. This can be achieved by confirming the correct location of the catheter and/or guidewire within the CBD prior to contrast injection and providing methods of reducing mechanical manipulation to achieve correct cannulation.

### SUMMARY

Aspects of the disclosure, according to some embodiments thereof, relate to improved catheters and guidewires for performing endoscopic retrograde cholangiopancreatography (ERCP). More specifically, but not exclusively, aspects of the disclosure, according to some embodiments thereof, relate to a catheter having multiple lumens, including lumen probes (sensors) configured to detect/measure/sense conductivity and/or resistivity and/or impedance and/or an electrical signal at an internal body cavity, to detect or determine if the distal end of the catheter is located in the bile duct or pancreatic duct, based on the differences in conductivity and resistivity between the contents of the ducts.

Aspects of the disclosure, according to some embodiments thereof, further relate to a guidewire, having a magnetic and/or ferromagnetic tip, capable of aiding in aiming the distal end (tip) of the guidewire into the common bile duct (CBD). In some embodiments, a magnetic force (exerted by an electromagnet placed externally to the subject body and/or by an electromagnet located on the distal part of an endoscope, through which the guidewire is passed and /or located on the distal part of a catheter through which the guidewire is passed) aids in bending the tip of the guidewire to be directed to the CBD rather than to the pancreatic duct. In some embodiments, the guidewire may further include one or more sensing elements, configured to detect/measure/sense conductivity and/or resistivity and/or impedance at an internal body cavity, to detect or determine if the distal end of the guidewire is located in the bile duct or pancreatic duct, based on the differences in conductivity and/or resistivity and/or impedance between the two ducts, as well as to optionally identify an inflammatory condition in the ducts.

According to some embodiments, the improved endoscopic retrograde cholangiopancreatography (ERCP) catheter system and/or guidewires provided herein and methods of using the same, advantageously allow minimizing the risk involved with the ERCP procedure and reduce its complications and side effects, such as, for example, Post-ERCP Pancreatitis (PEP), and optionally, bleeding, bowel holes and infections. In some embodiments, the improved devices and methods allow the performing of ERCP procedures, preferably, without inadvertent injection of contrast material into the pancreatic duct.

According to some embodiments, the devices (catheters and/or guidewires) and method provided herein allow avoiding mechanical manipulation and the undesired dye injection into the pancreatic duct, by advantageously allowing directing the guidewire into the bile duct, if incorrect placement is detected.

According to some embodiments, the devices and method provided herein are advantageous as they provide one or more of: a magnetically biased guidewire that has a higher probability of being inserted into the CBD (and not the pancreatic duct); a catheter and/or guidewire that can detect correct/incorrect insertion into the ducts; and/or if a change of location is required, the same catheter can direct the guidewire into the correct duct (i.e., CBD).

Thus, according to an aspect of some embodiments, there is provided an endoscopic retrograde cholangiopancreatography (ERCP) catheter which includes:
an elongated catheter body having a multiplicity of internal lumens, the lumens extending from a proximal end of the elongated catheter body to a distal end of the elongated catheter body opposite thereto, the distal end is configured to be positioned within the common bile duct (CBD) or the pancreatic duct of a subject, the lumens include:
one or more sensor lumens having (or configured to have) within the lumen one or more sensing probes extending from a proximal end of the lumen to a distal end of the lumen, said one or more probes are configured to sense or measure one or more of: electrical impedance, resistance, conductance and/or electrical signal, at the distal end of the catheter; and
a first lumen having an opening at a tip on the distal end of the catheter, said first lumen is configured to allow passage therethrough of a guidewire; and
a second lumen configured to allow passage of a guidewire therethrough, wherein the opening of the distal end of said second lumen is located at a proximal position relative to the opening of the first lumen, thereby allowing a guidewire passing through the second lumen to be positioned at a different internal body location compared to a guidewire passing through the first lumen.

In some embodiments, the sensing probes may include a conductive material configured to pass electrical current therethrough. In some embodiments, a sensor system located at the proximal end of the catheter or connected to the catheter, is configured to determine the resistance and/or conductance and/or impedance in the body cavity in which the distal end of the catheter is located, via said probes, and to assess the conductivity in said body region. In some embodiments, the electrical impedance and/or resistance and/or conductance values determined at the distal end of the catheter are indicative of the location of the distal end of the catheter to be in the bile duct or in the pancreatic duct. In some embodiments, wherein the higher the resistivity and/or impedance values and/or the lower the conductivity value that are measured at the distal end of the catheter, is indicative that the distal end of the catheter is located in the bile duct.

In some embodiments, the electrical impedance and/or resistance and/or conductance and/or electrical signal values determined at the distal end of the catheter are indicative of an inflammatory condition in the bile duct or in the pancreatic duct.

In some embodiments, the electrical signal is indicative of muscle or nerve activity. According to some embodiments, the electrical signal is configured to allow Electromyography measurements.

In some embodiments, the catheter may be configured to be passed through an endoscope. In some embodiments, the endoscope may be a duodenal endoscope (duodenoscope).

In some embodiments, the catheter may be sized to be passed through the duodenal endoscope, through the major duodenal papilla and Sphincter of Oddi and into the ampulla of Vater of the subject.

In some embodiments, the endoscope may include an electromagnetic or magnetic cuff/ region at the external circumference or at an internal cavity of the endoscope, preferably at a distal end thereof. In some embodiments, the magnetic region is located in the gastrointestinal tract. In some embodiments, the magnetic region includes an electromagnet that may be controlled, for example, remotely (i.e. externally to the body), by a health care provider. For example, the electromagnet may be turned off after entering the ducts, for example, the bile duct.

In some embodiments, the catheter may be made of a biocompatible and/or flexible material.

In some embodiments, the catheter may include at least two sensor lumens each having (configured to have) within the lumen a sensing probe extending from a proximal end of the lumen to a distal end of the lumen.

In some embodiments, the distal end of the second lumen is positioned at a proximal side region of the distal end of the catheter. In further embodiments, the location of the opening of the distal end of the second lumen within the subject body is controlled by rotating the catheter or at least a portion of the distal end of the catheter. In some embodiments, the second lumen may include an obstacle, selected from a bump, a distortion in shape of the lumen, a bridge or combinations thereof, located in proximity with the distal opening of said second lumen, configured to aid in directing a guidewire passing through the second lumen, to a desired location.

In some embodiments, the catheter may be a sphincterotomy catheter (with a mechanism that enables incision of the sphincter of Oddi).

In some embodiments the catheter may further include a magnetic region on the body of the catheter.

In some embodiments, the first and/or second lumens are configured to allow passage of one or more suitable medical instruments and/or substances. In some embodiments, the one or more suitable medical instruments and/or substances may be selected from: stent systems, cytology sheaths, dilators balloons, stent extractors, miniscopes, contrast dye, medication, or any combination thereof.

In some embodiments, the proximal region of the catheter includes one or more handles or ports for operating, inserting and/or maneuvering the guidewire, the medical instruments and/or substances.

In some embodiments the guidewire for use with the catheter may include a flexible, biocompatible material.

In some embodiments, the guidewire may further include comprises a magnetic tip at the distal end thereof.

In some embodiments, the magnetic tip of the guidewire may be configured to allow bending or deforming the distal end of the guidewire, to aid in directing or navigating the guidewire to the bile duct.

According to some embodiments, there is provided an endoscopic retrograde cholangiopancreatography (ERCP) catheter which includes:
an elongated catheter body having:
a distal end configured to be positioned within the common bile duct (CBD) or the pancreatic duct of a subject; and
a multiplicity of internal lumens extending from a proximal end of the elongated catheter body to a distal end of the elongated catheter body opposite thereto, the lumens include at least:
a first lumen having an opening at a tip on the distal end of the catheter, said first lumen is configured to allow passage therethrough of a guidewire;
a second lumen configured to allow passage of a guidewire therethrough, wherein the opening of the distal end of said second lumen is located at a proximal position relative to the opening of the first lumen, thereby allowing a guidewire passing through the second lumen to be positioned at a different internal body location compared to a guidewire passing through the first lumen; and
one or more sensing probes positioned on and/or in the elongated catheter body, the one or more sensing probes being configured to sense or measure one or more of: electrical impedance, resistance, conductance and/or electrical signal, at the distal end of the catheter.

According to some embodiments, one or more of the sensing probes extend from a proximal end of the catheter body to a distal end of the catheter body.

According to some embodiments, wherein one or more of the sensing probes may be positioned within one or more internal sensor lumens, said one or more internal sensor lumens extend from a proximal end of the elongated catheter body to a distal end of the elongated catheter body.

According to some embodiments one or more of the sensing probes may be positioned on an external circumference of the catheter body.

According to some embodiments, one or more of the sensing probes may be positioned on or at the distal end of the catheter. According to some embodiments one or more of the sensing probes is positioned on the tip of the distal end of the catheter.

According to some embodiments, there is provided a method for determining the location and/or for the positioning of a distal end of the catheter and/or guidewire, for preforming ERCP in a subject in need thereof, the method comprising: inserting a guidewire through the first lumen of the catheter, to protrude from the distal end of the catheter, wherein the catheter is inserted through an endoscope, through the major duodenal papilla and/or Sphincter of Oddi and/or into the ampulla of Vater of the subject; sensing, measuring and/or determining the conductivity and/or resistivity and/or impedance and/or electrical signal (electrical activity) at the body cavity in which the external end of the catheter and/ or guidewire is located; determining based on the conductivity and/or resistivity and/or impedance if the catheter and/or guidewire end is in the pancreatic duct or the bile duct; and if the catheter and/or guidewire tip is in the pancreatic duct, insertion of a guidewire through the second lumen of the catheter, such that the distal end of the guidewire inserted through the second lumen is positioned within the bile duct.

In some embodiments, the method may further include passing an electrical current through the probes of the catheter and/or guidewire. In some embodiments, the method may include determining the resistance and/or impedance and/or conductivity in the body cavity in which the distal end of the catheter and/or guidewire is located is performed using a sensor system located at the proximal end of the catheter and/or guidewire, or an external control unit to which the catheter and/or guidewire are connected. In some embodiments, the higher the resistivity and/or impedance values and/or the lower the conductivity value that are measured at the distal end of the catheter, it is indicative that the distal end of the catheter is located in the bile duct. In some embodiments, the method may include determining an inflammatory condition of the ducts, based on one or more of the measurements.

In some embodiments, the method may further include inserting one or more medical instruments or substances through a lumen of the catheter into the internal body cavity.

In some embodiments, the method may further include operating and/or maneuvering the guidewire, one or more medical instruments and/or substances within the bile duct.

In some embodiments, the method may further include applying an external magnetic force to cause the distal end of a guidewire comprising a magnetic and/or ferromagnetic tip to bend towards the bile duct.

According to some embodiments, there is provided a guidewire for use in ERCP procedure, said guidewire is made of or coated with a non-magnetic material and having a magnetic region at the distal end of the guidewire, said distal end is flexible, wherein said guidewire is configured to advance through a duodenal endoscope through the major duodenal papilla and/or Sphincter of Oddi and/or into the ampulla of Vater of the subject, wherein the magnetic tip of the guidewire is capable of mediating the distal end of the guidewire to bend in the direction of the bile duct of the subject, wherein the bending of the distal end of the guidewire is affected by an external magnetic force.

In some embodiment, the magnetic region at the distal end is made of a magnetic or ferromagnetic material or is coated with magnetic or ferromagnetic material.

In some embodiments, the guidewire may be configured to pass therethrough the endoscope within a lumen of a catheter, said catheter is passed within the lumen of the endoscope.

In some embodiments, the external magnetic force may be exerted by an electromagnet placed externally to the subject's body, or placed internally on the catheter and/or on the endoscope and/or within the endoscope. In some embodiments, the position and/or direction and/or intensity of the electromagnet is controlled, such as to the direct the magnetic force in the direction of the bile duct, to thereby cause the bending of the distal end of the guidewire located within the subject body in the direction of the bile duct. In some embodiments, the external magnetic force is exerted by a magnetic region/magnetic cuff positioned at the external circumference or in the internal lumen of the endoscope, at a distal end thereof.

In some embodiments, the guidewire may further include one or more sensing elements/probes, configured to sense, measure and/or detect electrical resistivity and/or impedance and/or conductivity at a body region in which the distal end of the guidewire is located. In some embodiments, the sensing elements comprise a conductive material, configured to pass electric current. In some embodiments, the sensing elements (sensors) may be positioned at the distal end of the guidewire or on the sides of the distal end of the guidewire. In some embodiments, the sensing elements (sensors) may be positioned coaxially or in parallel to each other, along external or internal length of the guidewire.

According to some embodiments, there is provided a method of aiming a guidewire into the bile duct of a subject, for preforming ERCP procedure, the method includes: inserting the guidewire through an endoscope and/or through a catheter, through the major duodenal papilla and/or Sphincter of Oddi and/or into the ampulla of Vater of the subject; and applying a magnetic force, such that the magnetic force causes the flexible distal end of the guidewire to bend in the direction of the bile duct.

In some embodiments, the external magnetic force may be exerted by an electromagnet placed externally to the subject's body. In some embodiments, the magnetic force may be exerted by a magnetic region of the catheter and/or the endoscope. In some embodiments, the magnetic region may be on the external surface of the endoscope of and/or within the internal lumen of the endoscope. In some embodiments, the magnetic region of the endoscope may be made of or coated with a magnetic or ferromagnetic material. In some embodiments, the magnetic region of the catheter may be made of or coated with a magnetic or ferromagnetic material.

In some embodiments, the method may further include a step of detecting, measuring and/or determining the electrical resistivity and/or impedance and/or conductivity and/or electrical activity at a body region in which the distal end of the guidewire is located.

Certain embodiments of the present disclosure may include some, all, or none of the above advantages. One or more other technical advantages may be readily apparent to those skilled in the art from the figures, descriptions, and claims included herein. Moreover, while specific advantages have been enumerated above, various embodiments may include all, some, or none of the enumerated advantages.

Unless otherwise defined, all technical and scientific terms used herein have the same meaning as commonly understood by one of ordinary skill in the art to which this disclosure pertains. In case of conflict, the patent specification, including definitions, governs. As used herein, the indefinite articles "a" and "an" mean "at least one" or "one or more" unless the context clearly dictates otherwise.

### BRIEF DESCRIPTION OF THE FIGURES

Some embodiments of the disclosure are described herein with reference to the accompanying figures. The description, together with the figures, makes apparent to a person having ordinary skill in the art how some embodiments may be practiced. The figures are for the purpose of illustrative description and no attempt is made to show structural details of an embodiment in more detail than is necessary for a fundamental understanding of the disclosure. For the sake of clarity, some objects depicted in the figures are not to scale. In the Figures:
**Figure** 1 schematically depicts a perspective view of a catheter, according to some exemplary embodiments;
**Figure 2A** schematically depicts a perspective side view of a distal end of a catheter, according to some exemplary embodiments;
**Figure 2B** schematically depicts a perspective close up view of a distal end of a catheter, according to some exemplary embodiments;
**Figure 2C** schematically depicts a perspective top view of a cross section of the distal end of a catheter, according to some exemplary embodiments;
**Figure 3A** schematically depicts a perspective side view of a catheter, according to some exemplary embodiments;
**Figure 3B** schematically depicts a perspective close up view of a distal end of a catheter and a guidewire, according to some exemplary embodiments;
**Figure 3C** schematically depicts a perspective view of a distal end of a catheter, with and guidewires, according to some exemplary embodiments;
**Figure 4A** schematically depicts a perspective close up view of a distal end of a catheter and lumen switching mechanism, according to some exemplary embodiments;
**Figure 4B** schematically depicts a perspective close up view of a distal end of a catheter and lumen switching mechanism, according to some exemplary embodiments;
**Figure 5A** schematically depicts a perspective view of a guidewire having a magnetic end, according to some exemplary embodiments;
**Figure 5B** schematically depicts a perspective close up view of a distal end of an endoscope and a guidewire having a magnetic end, according to some exemplary embodiments;
**Figure 6A** schematically illustrates a distal end of a catheter and guidewire within the bile duct region of a subject, according to some exemplary embodiments;
**Figure 6B** schematically illustrates a distal end of a catheter and guidewire within the pancreatic duct region of a subject, according to some exemplary embodiments;
**Figure 6C** schematically illustrates a distal end of a catheter and guidewire inserted via the second lumen of the catheter, within the bile duct region of a subject, according to some exemplary embodiments;
**Figures 7A****-E-** schematically illustrate a perspective view of the distal end of guidewires, having sensors arrangements; according to some embodiments; and
**Figure 8** - bar graphs showing resistivity values of various fluids, as determined by using electrical sensors.

### DETAILED DESCRIPTION

The principles, uses and implementations of the teachings herein may be better understood with reference to the accompanying description and figures. Upon perusal of the description and figures present herein, one skilled in the art will be able to implement the teachings herein without undue effort or experimentation. In the figures, same reference numerals refer to same parts throughout.

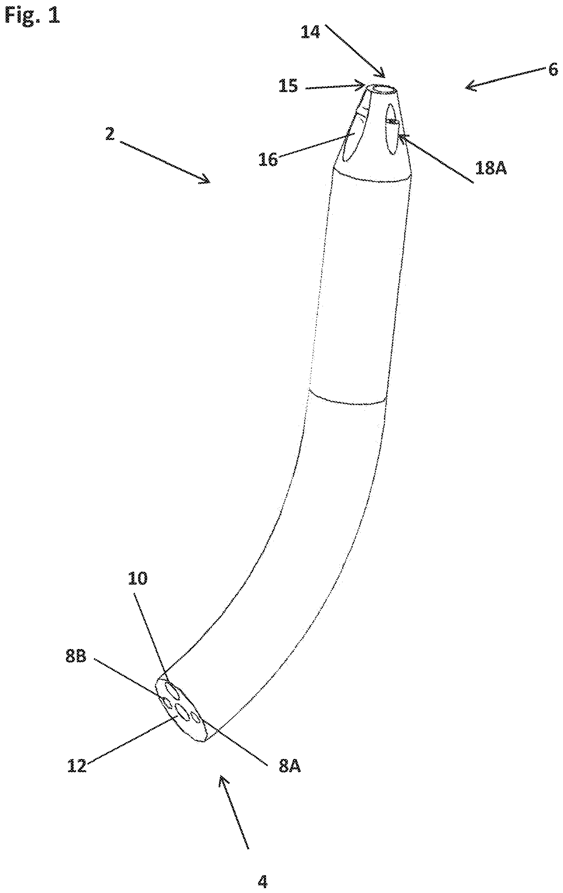

Reference is now made to **Fig. 1** which schematically depicts a perspective view of a catheter, according to some exemplary embodiments. As shown in Fig. 1, catheter 2, has a distal end (configured to be placed within a body cavity) and a proximal end (located externally to the subject body). The catheter can include several lumens that run along internal body of the catheter, from the proximal end to the distal end. The lumens include sensor lumens, extending from the proximal end (lumens 8A-8B) to the distal end (end of lumen 8B is shown as opening 18B), having probes/sensors running therethrough, which can detect/measure/sense the conductivity and/or resistivity and/or impedance at the region in which the proximal end of the catheter is located. The catheter may further include a first lumen (12), having an opening (14) at the tip (15) of the distal end of the catheter. The catheter may include a second lumen (10, having an opening (16) at a side region of the distal end of the catheter.

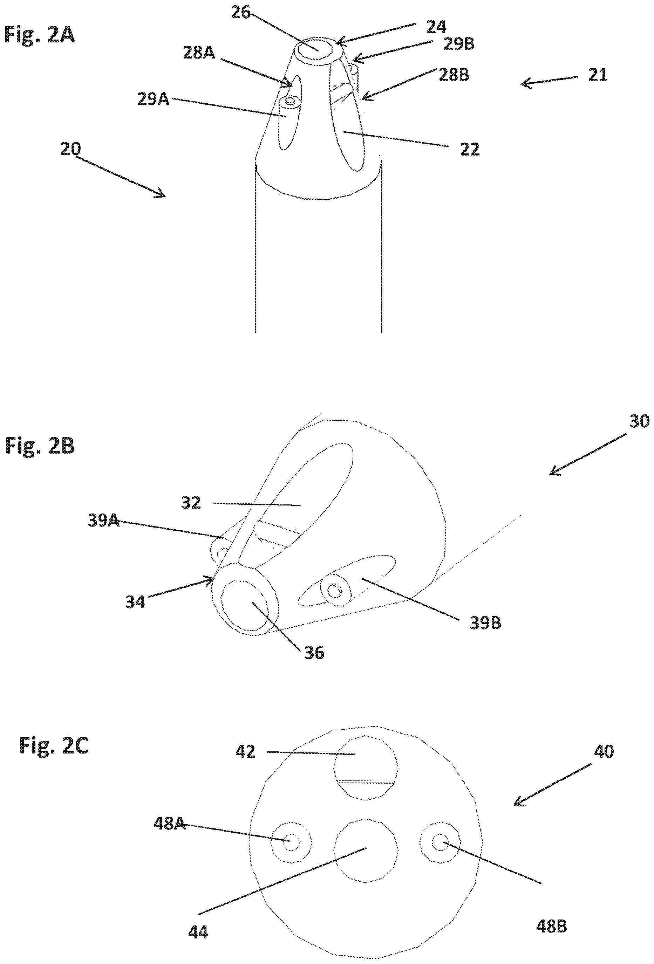

Reference is now made to **Fig. 2A**, which schematically depicts a perspective side view of a distal end of a catheter, according to some embodiments. As shown in Fig. 2A, distal end (21) of the catheter (20), has an opening (26) at the tip (24) of a first lumen. On a side region of the distal end, an opening (22) of a second lumen is located. Further shown are the tips (ends) of sensors 29A-B, which protrude through the openings (28A-B) of their respective lumens.

Reference is now made to **Fig. 2B**, which schematically depicts a perspective close up view of a distal end of a catheter, according to some embodiments. Shown in the close up view of Fig. 2B, is the distal end (30) of catheter, and the openings of the first lumen (36), located at the tip (34) of the distal end of the catheter. Further shown is the opening (32) of the second lumen and the ends of probes/sensors (39A-B). In some embodiments, the end of the second lumen has a bridge, a bump, a distortion, or an obstacle configured to allow bending of the tip of a guidewire passing through the second lumen.

Reference is now made to **Fig. 2C** which schematically depicts a perspective top view of a cross section of the distal end of a catheter, according to some embodiments. The top view of the distal end (40) of the catheter shows the opening of the first, central lumen (44), the openings of the sensor lumens (48A-48B) and the opening of the second, side lumen.

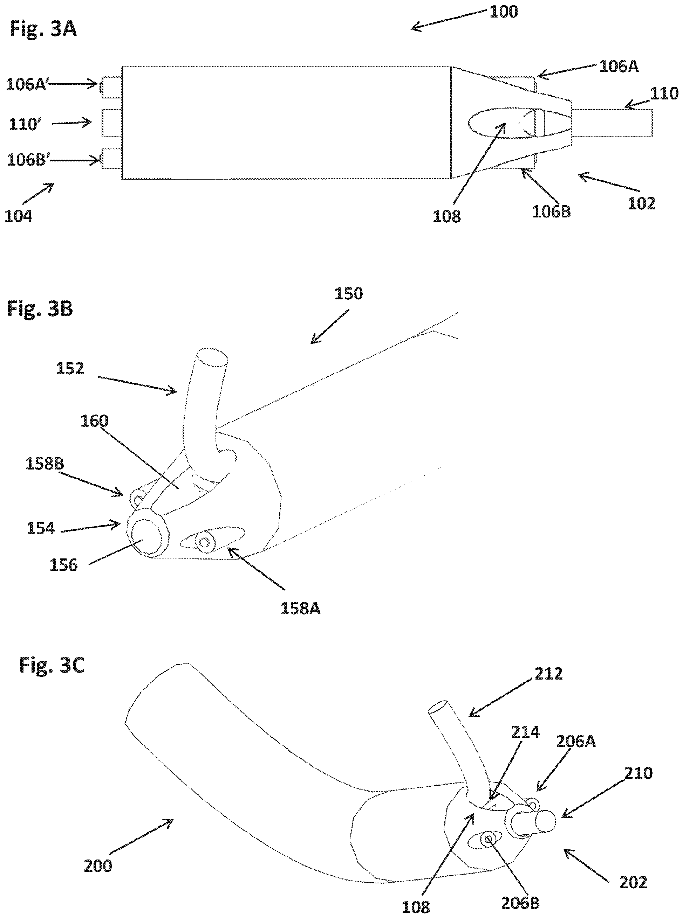

Reference is now made to **Fig. 3A**, which schematically depicts a perspective side view of a catheter and a guidewire protruding from the distal end of the first lumen of the catheter, according to some embodiments. As shown in Fig. 3A, catheter 100, has an elongated body, having a proximal end (104) and a distal end (102), configured to be placed within the subject body. The catheter includes several lumens extending from the proximal end to the distal end of the catheter. At the proximal end of the catheter, a proximal end of guidewire (110') is shown, the distal end of which (110) protrudes from the distal end of the first lumen of the catheter. Further, at the proximal end of the catheter, a proximal end of probes/sensors (106A'-B') is shown, the distal end of which (106A-B) protrudes from the distal end of the sensor lumens of the catheter. Further shown is the end (108) of the second lumen of the catheter, which is located at the side of the catheter distal end and is preferentially proximal to the tip of the catheter. The end of the second lumen may include a bump or distortion, to allow bending or guiding a guidewire or other suitable medical instrument protruding therethrough.

Reference is now made to **Fig. 3B**, which schematically depicts a perspective close up view of a distal end of a catheter and a guidewire protruding from the distal end of the second lumen, according to some embodiments. As shown in Fig. 3B, the distal end of catheter (150), has an opening (156) of a first lumen, located at the tip (154) of the distal end. The first lumen is shown empty (i.e., not having a guidewire protruding therethrough). The second lumen has a guidewire inserted therethrough, such that the distal end of the guidewire (152) protrudes from the opening (160) of the second lumen. Further shown at the distal end are the ends (tips) of sensors/probes (158A-B).

Reference is now made to **Fig. 3C**, which schematically depicts a perspective side view of a catheter having a first guidewire protruding from the distal end of the first lumen of the catheter and a second guidewire protruding from the distal end of the second lumen of the catheter, according to some embodiments. As shown in Fig. 3C, at the distal end (202) of catheter (200) shown is the distal end of guidewire (210) protruding from the distal end of the first lumen of the catheter. Further shown is the distal end of a second guidewire (212) protruding from the second lumen of the catheter. As shown in Fig. 3C, the second guidewire (212) is directed/aimed in a different direction as compared to the first guidewire. In some embodiments, the aiming/bending of the second guidewire may be achieved by an obstacle, a bent, a bump, a bridge or any other obstructing element (shown as element 214). The obstructing element may be made of any suitable biocompatible material, magnetic material, or be coated with a magnetic or ferromagnetic material. In some embodiments, the obstructing element may be integrally formed with the catheter body, or may be attached, placed, or mounted thereto. In some embodiments, the aiming/bending of the first and/or second guidewire may be achieved by a magnetic tip placed or formed with the distal end of the guidewire, as further detailed below. Further shown in Fig. 3C are the distal ends of sensors 206A-B.

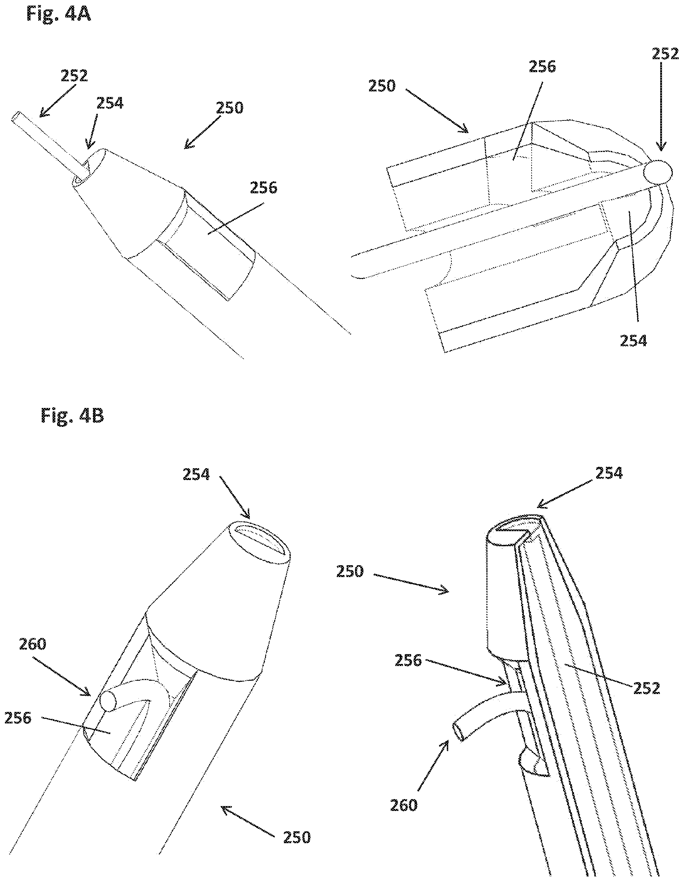

Reference is now made to **Fig. 4A**, which schematically depicts a perspective close up view of a distal end of a catheter and lumen switching mechanism, according to some embodiments. As shown in Fig. 4A, distal end of catheter 250 includes an opening of the first lumen (254), having a guidewire protruding therefrom (252). Further shown is the opening of the second lumen (256). On the right hand panel of Fig. 4A- a close up view of a cross section of the distal end is shown. Reference is made to **Fig. 4B**, which shows the distal end of catheter (250) which includes an opening of the first lumen (254). Further shown is the opening of the second lumen (256) having a guidewire (260) protruding therethrough.

According to some embodiments, the catheter may be made of any suitable biocompatible material. In some embodiments, the catheter may be at least partially flexible.

In some embodiments, the catheter may be a catheter that may function as a sphincterotome. In some embodiments, the catheter is a sphincterotomy catheter.

In some embodiments, the probes/sensors may include a conductive material configure to pass electrical current. In some embodiments, the probes may be made of any suitable metal.

According to some embodiments, a sensor system located at the proximal end of the catheter is configured to determine the resistance and/or conductance and/or impedance in the body cavity in which the distal end of the catheter is located, via the probes, and to assess the conductivity in said body region. In some embodiments, the electrical resistance and/or conductance and/or impedance values determined at the distal end of the catheter are indicative of the location of the distal end of the catheter in the common bile duct or in the pancreatic duct. In some exemplary embodiments, as exemplified below herein, the higher the resistivity and/or impedance value and/or the lower the conductivity value that are measured at the distal end of the catheter, it is indicative that the distal end of the catheter is located in the bile duct. In some exemplary embodiments, a resistivity value of over about 400 ohm-metre (Ωm), over about 500(Ωm), over about 550(Ωm), over about 600(Ωm), over about 700(Ωm), is indicative of a bile duct region.

According to some embodiments, a sensor system located at the proximal end of the catheter is configured to determine electrical signal or electrical activity, for example, of muscle fibers. In some embodiments, the sensor system may be configured to determine EMG activity in the body cavity in which the distal end of the catheter is located, via the probes.

According to some embodiments, the catheter system may be configured to detect or identify or recognize an inflammation or inflammatory condition in the ducts. In some embodiments, the identification of the inflammatory condition may be based at least in part on the electrical resistance and/or conductance and/or impedance and/or electrical activity measurements.

According to some embodiments, the lumen probes (sensors) disclosed herein may be configured to detect/measure/sense/determine one or more of: conductivity, resistivity, impedance and/or an electrical signal. Each possibility is a separate embodiment.

According to some embodiments, the lumen probes (sensors) disclosed herein may be configured to detect/measure/sense/determine one or more of: conductivity and/or resistivity and/or impedance and/or an electrical signal at an internal body cavity. Each possibility is a separate embodiment.

According to some embodiments, the catheter is configured to be passed through an endoscope, such as, a duodenal endoscope. In some embodiments, the catheter sis sized/constructed to be passed through the duodenal endoscope, through the major duodenal papilla and/or Sphincter of Oddi and/or into the ampulla of Vater of the subject. Each possibility is a separate embodiment.

According to some embodiments, the catheter may further include one or more additional internal lumens.

According to some embodiments, the catheter may include a magnetic portion or region along the body length of the catheter. In some embodiments, the magnetic portion of the catheter may be made of, or coated with a magnetic or ferromagnetic material. In some embodiments, the magnetic portion may be or include an electromagnet. In some embodiments the electromagnet may operate intermittingly (i.e., be turned on or off at will). In some embodiments, the operation of the electromagnet may be controlled externally, for example, by a health care provider operating the catheter system.

According to some embodiments, any of the lumens of the catheter (including the first/ second and/or any additional lumens) are configured to allow passage of one or more suitable medical instruments and/or substances. In some embodiments, the one or more suitable medical instruments and/or substances selected from: stent systems, sphincterotomes, cytology sheaths, dilators balloons, stent extraction devices miniscopes, contrast dye, medication, or any combination thereof. Each possibility is a separate embodiment.

According to some embodiments, the proximal region of the catheter may include one or more handles or ports for operating, inserting and/or maneuvering the guidewire, the medical instruments and/or substances.

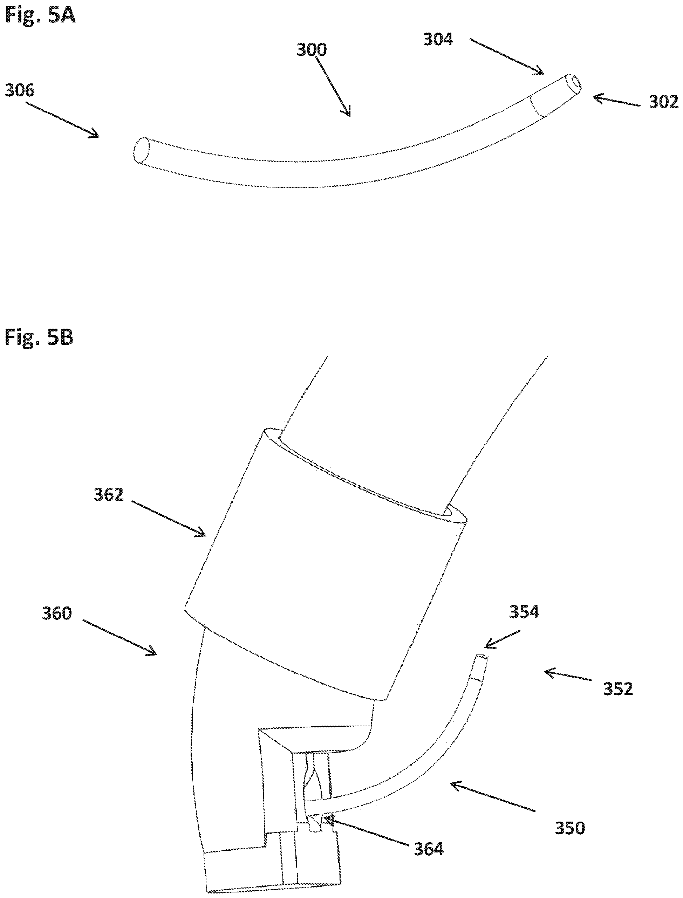

Reference is now made to **Fig. 5A**, which schematically depicts a perspective view of a guidewire having a magnetic end, according to some exemplary embodiments. As shown in Fig. 5A, the guidewire (300) has an elongated body having a proximal end (3026) and a distal end (302). The guidewire may include the distal end thereof, a magnetic tip/magnetic region (304). The magnetic tip of the guidewire is configured to allow mediating the distal end of the guidewire to bend in the direction of the common bile duct of a subject.

Reference is now made to **Fig. 5B**, which schematically depicts a perspective close up view of a distal end of an endoscope and a guidewire having a magnetic end, according to some embodiments. Shown in Fig. 5B, a distal end of an endoscope (36), having opening 364, through which guidewire (350) protrudes. The guidewire has a magnetic tip/region (354) at the distal end (352) thereof. Further shown is magnetic cuff 263, located on the circumference of the endoscope. The magnetic cuff can exert a magnetic force, capable of attracting the magnetic end of the guidewire and causing the distal end of the guidewire to bend, as illustrated in Fig. 5B.

According to some embodiments, the guidewire may be made of or coated with a non-magnetic material and having a magnetic region at the distal end of the guidewire. In some embodiments, the distal end of the guidewire may be flexible. In some embodiments, the body of the guidewire is substantially rigid or stiff. In some embodiments, the body of the guidewire is substantially flexible. In some embodiments, the guidewire is configured to advance through a duodenal endoscope. In some embodiments, the guidewire is configured to advance through a catheter lumen (wherein the catheter may be passed through an endoscope). In some embodiments, the guidewire is configured to be passed through an endoscope through the major duodenal papilla and/or Sphincter of Oddi and/or into the ampulla of Vater of a subject. In some embodiments, the magnetic region at the distal end of the guidewire may be made of a magnetic or ferromagnetic material or is coated with magnetic or ferromagnetic material. In some embodiments, the external magnetic force may be exerted by an electromagnet placed externally to the subject's body or placed internally on a catheter and/or on the endoscope and/or within the endoscope. According to some embodiments, the position direction and/or intensity of the electromagnet may be controlled such as to the direct the magnetic force in the direction of the bile duct of the subject, to thereby cause the bending of the distal end of the guidewire located within the subject body in the direction of the bile duct.

According to some embodiments, the guidewire may include one or more sensors/probes configured to sense/detect/measure electrical conductivity and/or resistivity and/or impedance and/or electrical signal/electrical activity. In some embodiments, the sensors are electrical sensors. In some embodiments, the electrical sensors may be positioned at the tip of the guidewire or on the sides of the distal end of the wire. In some embodiments, the electrical conducting probes/sensors/wires/cables/electrodes may be positioned coaxially (one around the other with insulation between them) or in parallel to each other, along the guidewire. In some embodiments, the guidewire may further be at least partially coated with different materials which provide desired properties such as, a hydrophilic coating.

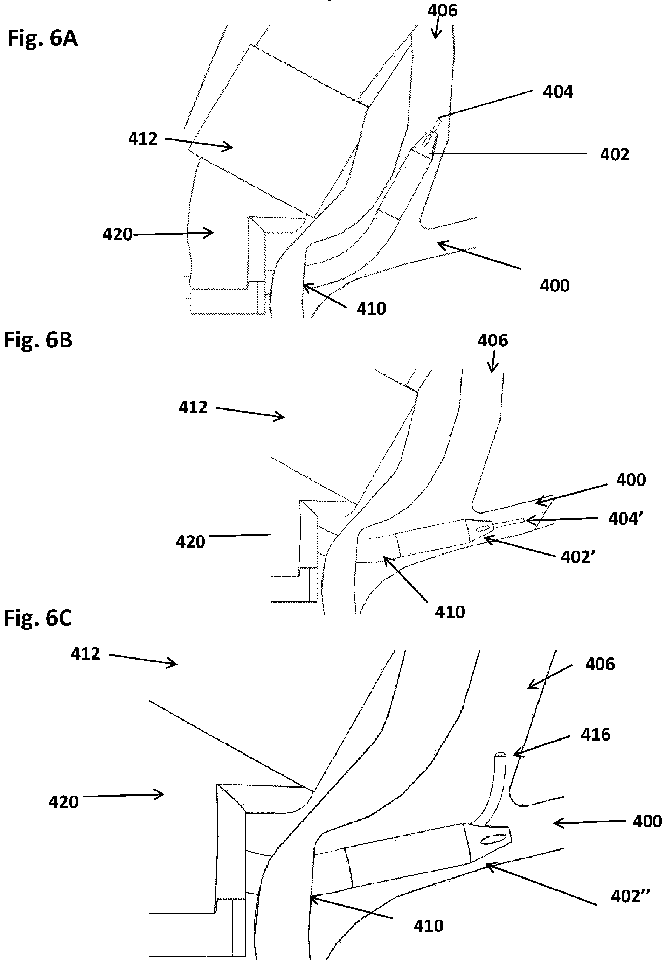

Reference is now made to **Fig. 6A** which schematically illustrates a distal end of a catheter and guidewire within the bile duct region of a subject, according to some embodiments. As shown in Fig. 6A, via endoscope 420, catheter 402 protrudes and guided into the bile duct (406), with the guidance of guidewire (404), protruding from the first lumen of the catheter. Further shown is pancreatic duct (400). The endoscope may include at a distal region thereof a magnetic cuff (412), which can aid is aiming/attracting a magnetic end of the guidewire towards the bile duct.

Reference is now made to **Fig. 6B** which schematically illustrates a distal end of a catheter and guidewire within the pancreatic duct region of a subject, according to some embodiments. As shown in Fig. 6B, via endoscope (420), catheter (402') protrudes and guided into the pancreatic duct (400), with the guidance of guidewire (404'), protruding from the first lumen of the catheter. Further shown is bile duct (406). The endoscope may include at a distal region thereof a magnetic cuff (412), which when exerting a magnetic force can aid is aiming/attracting a magnetic end of the guidewire.

Reference is now made to **Fig. 6C** which schematically illustrates a distal end of a catheter and guidewire inserted via the second lumen of the catheter, within the bile duct region of a subject, according to some embodiments. As shown in Fig. 6C, via endoscope (420), catheter (402") protrudes. The catheter has a guidewire (416) protruding through the second lumen of the catheter, such the guidewire distal end is located in the bile duct (406). Further shown is pancreatic duct (400). The endoscope may include at a distal region thereof a magnetic cuff (412), which when exerting a magnetic force can aid is aiming/attracting a magnetic end of the guidewire towards the bile duct.

In some embodiments, when the catheter (and guidewire) are inserted into the body cavity (for example, by being passed through an endoscope, via the major duodenal papilla and/or Sphincter of Oddi and/or into the ampulla of Vater of a subject), the probes at the distal end of the catheter (or in some embodiments, located on the guidewire), can be used to sense/measure/detect/determine the electrical conductivity/resistivity/impedance/activity at the distal region. If, based on the measurements it is determined that the distal end of the catheter (and/or guidewire) are placed in the bile duct (as illustrated in Fig. 6A), further procedures in the bile duct can be continued. If, based on the measurements it is determined that the distal end of the catheter (and/or guidewire) is rather placed in the pancreatic duct (as illustrated in Fig. 6B), further procedures are not commenced, until the catheter (or a guidewire) is redirected to common bile duct (CBD). This can be done by inserting a second guidewire, through a second lumen of the catheter, such that the second guidewire is biased to be placed in the bile duct (as illustrated in Fig. 6C). Alternatively, in some embodiments, the first guidewire is retracted and positioned in the second lumen of the catheter, such that is biased to be placed in the bile duct.

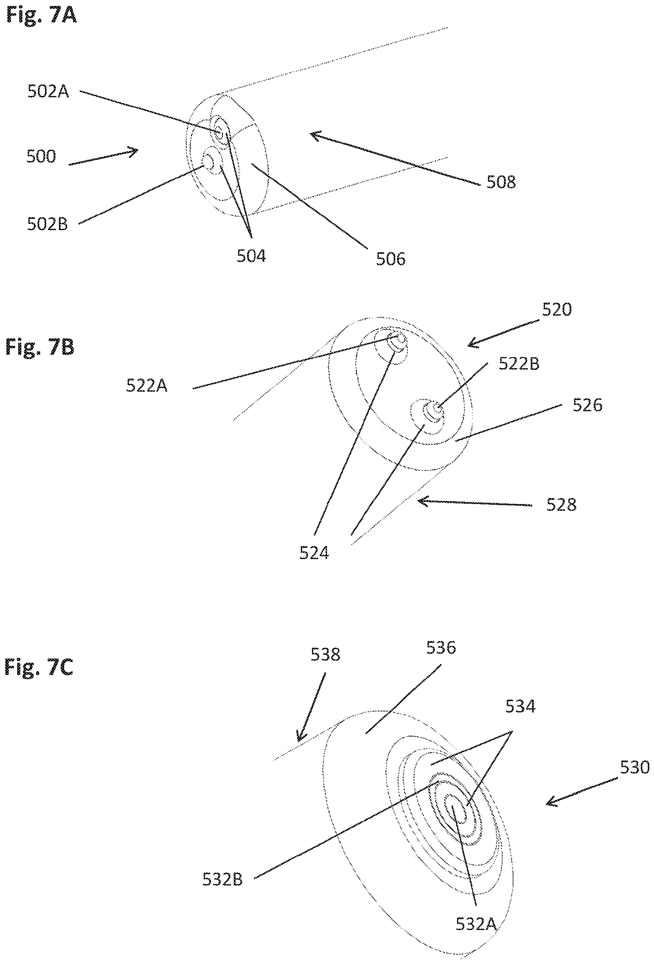

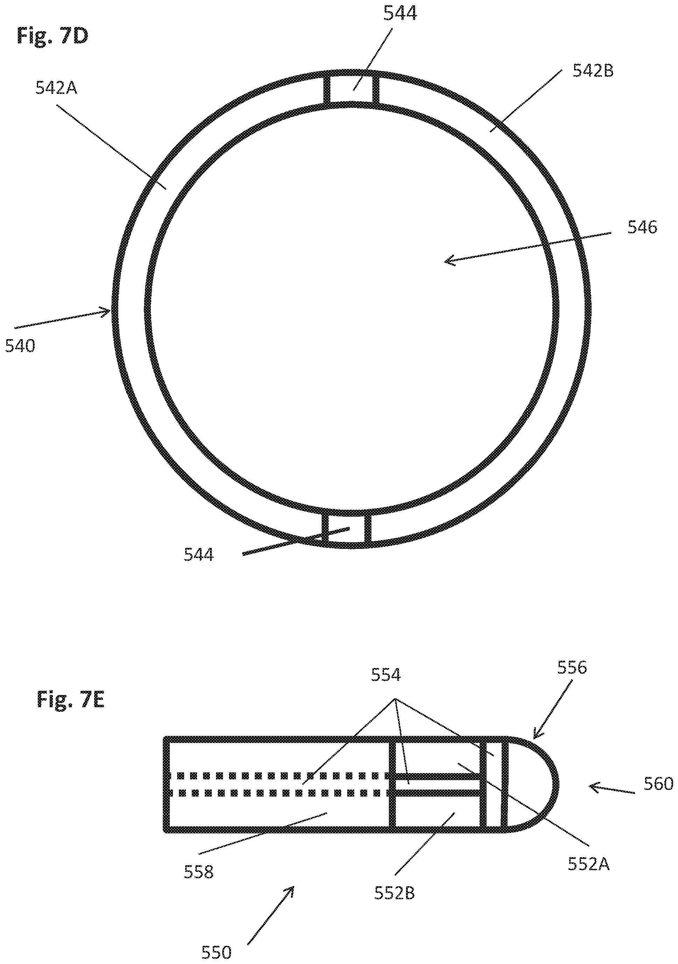

Reference is now made to Figs. 7A-E, which schematically illustrate perspective views of the distal end of guidewires, having sensors arrangements, according to some embodiments. **Fig. 7A** shows the end of guidewire, 500, having sensors (502A-B), arranged in parallel to each other and each passing along a different region of the guidewire. The sensors (502A and 502B) are arranged in parallel, and are insulated, so as to allow passage of electrical current. The sensors may run along the external or internal circumference of the guidewire. The insulation (504) is of and/or between the sensors. In some embodiments, the sensors are electrodes. Further shown is magnet (506), configured in aiding bending the tip of the guidewire (as detailed above). The guidewire may be further externally coated (coating 508), to aid operating the guidewire. **Fig. 7B** shows the end of guidewire, 520, having sensors (522A-B), arranged in parallel to each other and each passing along a different region of the guidewire. The sensors (522A and 522B) are arranged in parallel, and are insulated, so as to allow passage of electrical current. The insulation (524) is of and/or between the sensors. In some embodiments, the sensors are electrodes. Further shown is magnet (526), configured in aiding bending the tip of the guidewire (as detailed above). The guidewire may be further externally coated (coating 528), to aid operating the guidewire. **Fig. 7C** shows the end of guidewire, 520, having sensors (522A-B), arranged co-axially to each other and each passing along a different region of the guidewire. The sensors (532A and 532B) are arranged in coaxial design, and are insulated, so as to allow passage of electrical current. The insulation (534) is of and/or between the sensors. In some embodiments, the sensors are electrodes. Further shown is magnet (536), configured in aiding bending the tip of the guidewire (as detailed above). The guidewire may be further externally coated (coating 538), to aid operating the guidewire. **Fig. 7D** shows a top view of the end of guidewire, 540, having sensors (542A-B), arranged in parallel to each other and each passing along a different region of the guidewire. The sensors (542A and 542B) are arranged in parallel, and are insulated, so as to allow passage of electrical current. The insulation (544) is of and/or between the sensors. In some embodiments, the sensors are electrodes. Further shown is magnet (546) at the tip of the distal end of the guidewire, configured in aiding bending the tip of the guidewire (as detailed above). **Fig. 7E** shows a perspective side view of a guidewire, 550, having sensors (552A-B), arranged in parallel to each other and each passing along a different region of the guidewire. The sensors (542A and 542B) are arranged in parallel, and are insulated, so as to allow passage of electrical current. The insulation (544) is of and/or between the sensors. In some embodiments, the sensors are electrodes. Further shown is magnet (546) at the tip of the distal end (560) of the guidewire, configured in aiding bending the tip of the guidewire (as detailed above). The guidewire may be further externally coated (coating 528).

According to some embodiments, there is provide a method for determining and/or positioning the location of a distal end of the catheter, for preforming ERCP in a subject in need thereof, the method includes one or more of the steps of:
- inserting a guidewire through the first lumen of the catheter of, such that the distal end of the guidewire protrudes from the distal end of the catheter, wherein the catheter is inserted through an endoscope, through the major duodenal papilla and/or Sphincter of Oddi and/or into the ampulla of Vater of a subject of a subject;
- sensing, measuring and/or determining the conductivity and/or resistivity and/or impedance and/or electrical activity at the body cavity in which the external end of the catheter is located;
- determining, based on the conductivity or resistivity if the catheter end is in the pancreatic duct or the bile duct; and
- if the catheter end is in the pancreatic duct, insert a guidewire (a second guidewire or the same guidewire) through a second lumen of the catheter, such that the distal end of the guidewire inserted through the second lumen is positioned within the bile duct.

According to some embodiments, the methods may further include one or more of:
- passing an electrical current through the probes of the catheter.
- inserting one or more medical instruments or substances through a lumen of the catheter into the internal body cavity.
- operating and/or maneuvering the guidewire, one or more medical instruments and/or substances within the bile duct.
- optionally applying an external magnetic force to cause the distal end of a guidewire comprising a magnetic tip to bend towards the bile duct.

In some embodiments, the guidewire passing through the first lumen and/or the second lumen of the catheter is the same guidewire (for example, the guidewire may be retracted from the first lumen and re-inserted through the second lumen). In some embodiments, a first guidewire is configured to be inserted into the first lumen and a second guidewire may be inserted into the second lumen. In some embodiments, the first guidewire and the second guidewire may be identical, similar or different with respect of size (diameter, length), type, composition and/or shape. Each possibility is a separate embodiment.

According some embodiments, there is provided an ERCP catheter configured for determining the location and/or positioning of a distal end of the catheter within the CBD or the pancreatic duct (PD) of a subject, said catheter includes an elongated catheter body comprising a multiplicity of internal lumens, said lumens extend from a proximal end to the distal end opposite thereto, said distal end is configured to be positioned within the biliary tract; said lumens include:
at least two sensor lumens each having within the lumen a sensing probe extending from the proximal end of the lumen to the distal end of the lumen, said probes are configured to sense or measure electrical impedance and/or resistance and/or conductance at the distal end of the catheter; and
a first lumen having an opening at a tip on the distal end of the catheter, said first lumen is configured to allow passage therethrough of a guidewire; and
a second lumen configured to allow passage of a guidewire therethrough, wherein the opening of the distal end of said second lumen is located at a proximal position relative to the opening of the first lumen, thereby allowing a guidewire passing through the second lumen to be positioned at a different internal body location compared to the guidewire passing through the first lumen.

According to some embodiments, there is provided an endoscopic retrograde cholangiopancreatography (ERCP) catheter which includes:
an elongated catheter body having a multiplicity of internal lumens, the lumens extending through at least part of the length from a proximal end of the elongated catheter body to a distal end of the elongated catheter body opposite thereto, the distal end is configured to be positioned within the common bile duct (CBD) or the pancreatic duct of a subject, the lumens include:
a first lumen having an opening at a tip on the distal end of the catheter, said first lumen is configured to allow passage therethrough of a guidewire; and
a second lumen configured to allow passage of a guidewire therethrough, wherein the opening of the distal end of said second lumen is located at a proximal position relative to the opening of the first lumen, thereby allowing a guidewire passing through the second lumen to be positioned at a different internal body location compared to a guidewire passing through the first lumen.

In some embodiments, the ERCP catheter may further include one or more sensor lumens each having within the lumen a sensing probe at least partially extending from a proximal end of the lumen to a distal end of the lumen, the probes are configured to sense or measure one or more of: electrical impedance, resistance, conductance and/or electrical signal, at the distal end of the catheter.

According to some embodiments, there is provided a catheter with a magnet (such as, an electromagnet) that is placed more distal than the end of the catheter entering the duct. The magnet allows bending the tip of the guidewire to a sharp degree (in the range of for example, up to 90 degrees), thereby assisting the tip to fold backwards to a U shape and aids its navigation to the correct duct. According to some embodiments, once the tip is placed inside a duct a second verification step may be executed, to verify that it is in the correct duct (and not in the pancreatic duct, as undesired injection of contrast agent to this duct can lead to severe pancreatitis). This may be done by conductivity/resistivity and/or by impedance measurements using an electrode probe/sensor. Pancreatic juice contains bicarbonate buffer and thus it is more conductive. Bile salt are hydrophobic and are thus less conductive. Based on the conductivity difference, it is possible to measure the placement in the correct duct. In some embodiments, if identified to be in the correct duct- procedure may be continued. If the conductivity measurements indicate the catheter tip is in the incorrect pancreatic duct, then there is a second port (lumen) in the catheter, for inserting a second guidewire. As the incorrect pancreatic duct is already plugged by the misguided first guidewire, the second guidewire inserted can enter the bile duct.

As used herein, the terms "bile duct", "common bile duct", "CBD" and "biliary duct" may interchangeably be used.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. No feature described in the context of an embodiment is to be considered an essential feature of that embodiment, unless explicitly specified as such.

Although steps of methods according to some embodiments may be described in a specific sequence, methods of the disclosure may include some or all of the described steps carried out in a different order. A method of the disclosure may include a few of the steps described or all of the steps described. No particular step in a disclosed method is to be considered an essential step of that method, unless explicitly specified as such.

Although the disclosure is described in conjunction with specific embodiments thereof, it is evident that numerous alternatives, modifications and variations that are apparent to those skilled in the art may exist. Accordingly, the disclosure embraces all such alternatives, modifications and variations that fall within the scope of the appended claims. It is to be understood that the disclosure is not necessarily limited in its application to the details of construction and the arrangement of the components and/or methods set forth herein. Other embodiments may be practiced, and an embodiment may be carried out in various ways.

The phraseology and terminology employed herein are for descriptive purpose and should not be regarded as limiting. Citation or identification of any reference in this application shall not be construed as an admission that such reference is available as prior art to the disclosure. Section headings are used herein to ease understanding of the specification and should not be construed as necessarily limiting.

As used herein, the term "about" may be used to specify a value of a quantity or parameter (e.g. the length of an element) to within a continuous range of values in the neighborhood of (and including) a given (stated) value. According to some embodiments, "about" may specify the value of a parameter to be between 80 % and 120 % of the given value. For example, the statement "the length of the element is equal to about 1 m" is equivalent to the statement "the length of the element is between 0.8 m and 1.2 m". According to some embodiments, "about" may specify the value of a parameter to be between 90 % and 110 % of the given value. According to some embodiments, "about" may specify the value of a parameter to be between 95 % and 105 % of the given value.

In the description and claims of the application, each of the words "comprise" "include" and "have", and forms thereof, are not necessarily limited to members in a list with which the words may be associated.

### EXAMPLES

### Example 1: Determining resistivity values of physiological and other fluids

In order to determine the differences in electrical properties of various physiological and other fluids, the resistivity of different fluids, including bile juice, and compositions mimicking pancreatic environment (such as trypsin) was determined, using an electrical sensor.

The sensor uses two probes to pass current through the tested liquids, and then measures the resistance or impedance, from which conductivity can be calculated. This type of measurement assesses the concentration of ions in the solution, whereby the more ions that are in the solution, the higher the conductivity.

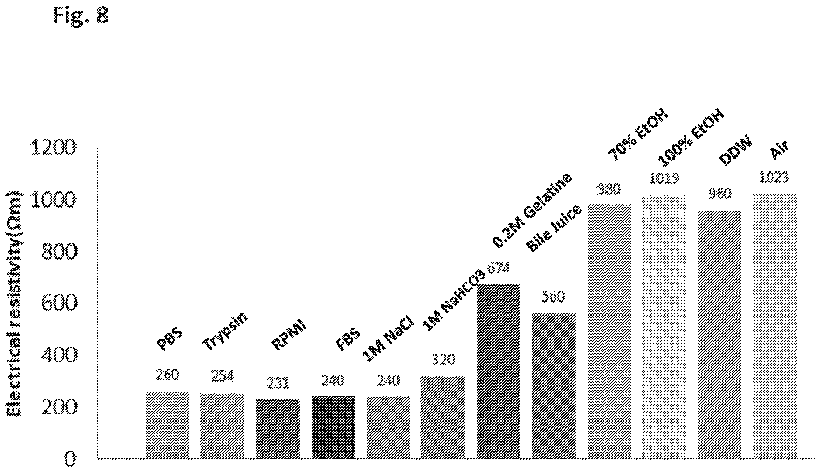

The results are presented in **Fig. 8**, which clearly demonstrate a difference in resistivity and conductivity between Ovis aries (sheep) bile juice and Trypsin solution which represents the pancreatic juice.

While a number of exemplary aspects and embodiments have been discussed above, those of skill in the art will recognize certain modifications, permutations, additions and sub-combinations thereof. It is therefore intended that the following appended claims and claims hereafter introduced be interpreted to include all such modifications, permutations, additions and sub-combinations as are within their true spirit and scope.

## Claims

1. An endoscopic retrograde cholangiopancreatography (ERCP) catheter system comprising:
a guidewire;
an elongated catheter comprising:
a distal end configured to be positioned within the common bile duct (CBD) or the pancreatic duct of a subject; and
a multiplicity of internal lumens extending from a proximal end of the elongated catheter to a distal end of the elongated catheter opposite thereto, said lumens comprising at least a first lumen having an opening at a tip on the distal end of the catheter, said first lumen is configured to allow passage therethrough of the guidewire; and
one or more sensors configured to sense or measure one or more of: electrical impedance, resistance, conductance and electrical signal, at the distal end of the catheter and/or the guidewire.

2. The catheter system according to claim 1, wherein the one or more sensors are positioned on and/or in the elongated catheter and/or the guidewire.

3. The catheter system according to either one of claims 1-2, wherein the sensors are positioned at the distal end of the guidewire or on the sides of the distal end of the guidewire.

4. The catheter system according to any one of claims 1-3, wherein the one or more sensors comprise a conductive material configured to pass electrical current therethrough.

5. The catheter system according to any one of claims 1-4, further comprising a sensor system located at the proximal end of the catheter, said sensor system is configured to determine the resistance and/or impedance and/or conductance in a body cavity in which the distal end of the catheter and/or the guidewire is located, via said sensors.

6. The catheter system according to any one of claims 1-5, wherein the electrical impedance and/or resistance and/or conductance values determined at the distal end of the catheter and/or the guidewire are indicative of the location of the distal end of the catheter and/or the guidewire in the common bile duct or in the pancreatic duct of the subj ect.

7. The catheter system according to any one of claims 1-6, wherein the higher the resistivity and/or impedance values and/or the lower the conductivity value measured at the distal end of the catheter and/or the guidewire are, the more indicative it is that the distal end of the catheter and/or the guidewire is located in the common bile duct.

8. The catheter system according to any one of claims 1-7, wherein the catheter system is configured to detect an inflammatory condition in the common bile duct or the pancreatic duct based, at least in part, on the sensor measurements.

9. The catheter system according to any one of claims 1-8, wherein the electrical signal is indicative of muscle or nerve activity.

10. The catheter system according to any one of claims 1-8, wherein the electrical signal is configured to allow Electromyography measurements.

11. The catheter system according to any one of claims 1-10, wherein the catheter is sized to be passed through a duodenal endoscope, through the major duodenal papilla and/or Sphincter of Oddi and/or into the ampulla of Vater of the subject.

12. The catheter system according to any one of claims 1-11, wherein the multiplicity of internal lumens are configured to allow passage of one or more suitable medical instruments and/or substances.

13. The catheter system according to claim 12, wherein the one or more suitable medical instruments and/or substances are selected from: stent systems, cytology sheaths, dilators balloons, stent extractors, miniscopes, contrast dye, medication, or any combination thereof.
